# EUROPEAN PATENT SPECIFICATION

(11) **EP 1 369 113 B1**
(45) Date of publication and mention of the grant of the patent: **22.11.2006**
(21) Application number: 02012602.5
(22) Date of filing: 06.06.2002
(51) Int. Cl.: A61K 9/72, A61K 9/107, A61K 9/00

(54) **Solubilisation of drugs in HFA propellant by means of emulsions**
Lösung von Wirkstoffen in HFA-Treibgasen mittels Emulsionen
Solubilisation de médicaments à l'aide d'émulsions dans les propulseurs HFA

(43) Date of publication of application: 10.12.2003
(73) Proprietor: CHIESI FARMACEUTICI S.p.A., I-43100 Parma (IT)
(72) Inventor: Meakin, Brian John, 43100 Parma (IT); Lewis, David Andrew, 43100 Parma (IT); Berrill, Susan Ann, 43100 Parma (IT); Davies, Rebecca Jayne, 43100 Parma (IT)
(74) Representative: Minoja, Fabrizio

(56) References cited:
- EP-A- 1 066 829
- EP-A- 1 080 720
- WO-A-00/37051
- WO-A-01/66085
- WO-A-01/72278
- WO-A-01/78689
- WO-A-01/87268
- WO-A-98/56349
- WO-A-02/066008
- US-A1- 2001 046 474
- US-B1- 6 221 378
- US-B1- 6 231 882
- US-B1- 6 312 665

## Description

The present invention relates to a water-in-oil emulsion or microemulsion formulations in HFA propellant systems to be administered through pressurized Metered Dose Inhalers (pMDIs). The invention also relates to oil-in-water emulsion formulations and provides methods for the preparation of the formulations.

Pharmaceutically active compounds could be administered to the respiratory tract by using pressurised metered dose inhalers (pMDIs). pMDIs use a propellant to expel droplets containing the pharmaceutical product to the respiratory tract as an aerosol.

As far as the type of propellant is concerned, hydrofluoroalkanes [(HFAs) also known as hydro-fluoro-carbons (HFCs)] would be mandatory propellants as chlorofluorocarbons (known also as Freons or CFCs), which were for many years the preferred propellants aerosols for pharmaceutical use, have been implicated in the destruction of the ozone layer so their use is being phased out. In particular, 1,1,1 ,2-tetrafluoroethane (HFA 134a) and 1,1,1,2,3,3,3-heptafluoropropane (HFA 227) have been acknowledged to be the best candidates for non-CFC propellants and a number of pharmaceutical aerosol formulations using such HFA propellant systems have been disclosed.

An aerosol pharmaceutical formulation in HFA propellant can be a solution or a suspension. Solution formulations, with respect to suspensions, do not present problems of physical stability of the suspended particles and so could guarantee a higher dose uniformity and reproducibility.

When the formulation is in the form of suspension, the particle size of the cloud is dominated by the particle size of the suspended drug, defined by the milling/micronization process.

When the formulation is in the form of solution, the volumetric contribution of suspended drug particles is absent and much finer liquid droplets clouds, largely defined by the drug concentration in the solution, are generated.

The aerosol formulations in solution offer the advantage of being homogeneous with the active ingredient and excipients completely dissolved in the propellant vehicle or its mixture with suitable co-solvents such as ethanol. Solution formulations also obviate physical stability problems associated with suspension formulations so assuring reproducible delivering of the dose.

Aerosol solution formulations in HFA known from the prior art generally contemplate the use of a cosolvent. The preferred cosolvent is ethanol. The PCT Applications WO 92/06675 and WO 95/17195 describe aerosol formulations respectively comprising as active ingredient beclomethasone 17,21-dipropionate or flunisolide in HFA 134a, HFA 227 or their mixtures and ethanol in an amount effective to solubilise the active ingredient in the propellant.

Despite their advantages with respect to suspensions, also solution formulations present some drawbacks such as chemical stability problems of the active ingredient in the propellant and/or in the propellant/cosolvent system.

Alternative methods of solubilisation of drugs in pMDIs have been reported in the literature. For example Evans and Farr in US 5292499 patented a propellant based medical aerosol formulation in which the drug is dissolved in reverse micelles. The preferred surfactant for this formulation is phosphatidyl choline (0.025 - 2.5 % w/v) and the resulting formulation appears to be a homogeneous solution. Analogous formulations of proteins and peptides (i.e. insulin) in reverse micelles have also been claimed in US 5230884 wherein the preferred surfactants are phospholipids, sorbitan mono- and tri-oleates, diolein, oleic acid.

Reverse (polar liquid-in-fluorocarbon) emulsion and reverse microemulsion composition in a fluorocarbon continuous phase for the delivery of polar liquid-soluble drugs have been described by Alliance in WO96/40057. These systems comprise a disperse aqueous phase containing polar drugs or diagnostic agents, a continuous phase comprising at least a one fluorocarbon and at least one nonfluorinated surfactants. The pulmonary administration of these systems is via liquid ventilation using a delivery device selected from endotracheal tube, intrapulmonary catheter, and a nebuliser and no references are given on the administration in pMDIs with hydrofluoroalkane propellants.

Generex described mixed micellar pharmaceutical formulations in a HFA propellant directed to a proteinic pharmaceutical agent also comprising a phenol (WO 00/37052) or compounds selected from the group consisting of lecithin, hyaluronic acid, glycolic acid, lactic acid and others either as micelle forming compounds (WO 00/37051) or absorption ehancing compounds (WO 01/15666).

An emulsion is a thermodynamically unstable system consisting of at least two immiscible liquid phases, one of which is dispersed as globules in the other liquid phase. The system is stabilized by the presence of an emulsifying agent or surfactant. The particle diameter of the dispersed phase generally extends from about 0.1 to 10 µ, although particle diameters as small as 0.01 µ, and as large as 100 µ are not uncommon in some preparations.

The size of microemulsion droplets is generally in the range of 0.006-0.02 µ (6-20 nm).

The type of emulsion which is produced, oil-in water (o/w) or water-in-oil (w/o), depends primarily on the property of the emulsifying agent. This characteristic is referred to as the hydrophilic-lipophilic balance, i.e. the polar-non polar nature of the emulsifier. Whether a surfactant is an emulsifier, wetting agent, detergent or solubilizing agent may be predicted from a knowledge of the hydrophile-lipophile balance. The type of emulsion is a function of the relative solubility of the surface active agent, the phase in which it is more soluble being the continuous phase. This is sometimes referred to as the rule of Bancroft, who observed the phenomenon in 1913. Thus, an emulsifying agent with a high HLB is preferentially soluble in water and results in the formation of an o/w emulsion. The reverse situation is true with surfactants of low HLB which tend to form w/o emulsions.

It has now been found that it is possible to prepare emulsion and microemulsion aerosol formulations to be delivered through pMDIs, using HFA propellants as the oil phase and incorporating the drug into the internal aqueous phase. Therefore the present invention provides a method of solubilising hydrophilic drugs in HFA propellant systems, by preparing a water-in-oil emulsion or microemulsion pMDI formulations. The drug shall be preferably a hydrophilic drug.

The formulation of the invention consists in a water-in-oil emulsion and microemulsion whereby the drug is preferably a hydrophilic drug and is incorporated into the internal aqueous phase and the HFA propellant is the external oil phase.

The invention further provides a method for the preparation of oil-in-water emulsion formulations.

The formulation comprises:
a) an effective amount of a medicament
b) a hydrofluoroalkane propellant selected from the group of HFA 134a, HFA 227 and their mixtures
c) one or more surfactants
d) small amounts of water and
e) optionally a cosolvent.

Suitable medicaments for the aerosol formulation according to the invention are fundamentally all active ingredients compounds which can be administered as aerosol through the oral and nasal membranes or respiratory tract. Both the oral and nasal membranes offer advantages over other routes of administration. For example, drugs administered through these membranes have a rapid onset of action, provide therapeutic plasma levels, avoid first pass effect of hepatic metabolism. The delivering to the lungs allows the medicament be absorbed into the blood stream via the lungs to obtain a systemic effect. Examples of suitable medicaments are beta-mimetics, corticosteroids, anticholinergics, cyclooxigenase-, mast cell-, lipoxigenase- and proteolytic enzyme - inhibitors, arachidonic acid-, leukotriene-, thromboxane-, sodium/potassium channel-, neurokinin-, tachykinin-, bradykinin-, muscarine-, histamine-, phosphodiesterase- and selectin - antagonists, potassium channel blockers, anti-infective agents, antibiotics, pentamidine, cytostatics, fungistatics, free-radical scavengers, vitamins, hormones, immunostimulants, immunosuppresssants, heparin, antidiabetics, analgesics, hypnotics and the like, for example:
beta-mimetics such as salbutamol, formoterol, salmeterol, TA 2005, fenoterol, clenbuterol, terbutaline, bambuterol, broxaterol, ephedrine, epinephrine, phenylephrine, isoprenaline, isoetharine, metaproterenol, orciprenaline, hexoprenaline, pirbuterol, tulobuterol, reproterol, rimiterol, bamethan, etc.,
corticoids such as beclomethasone, betamethasone, ciclomethasone, dexamethasone, triamcinolone, budesonide, butixocort, ciclesonide, fluticasone, flunisolide, icomethasone, mometasone, tixocortol, loteprednol, tipredane, etc.,
anticholinergics and spasmolytics such as atropine, scopolamine, N-butylscopolamine, ipratropium bromide, oxitropium bromide, thiotropium bromide, drofenine, oxybutinine, moxaverine, glycopyrrolate, etc.,
mast cell inhibitors such as cromoglycic acid, nedocromil, etc.,
lipoxygenase inhibitors such as zileuton,
leukotriene antagonists such as iralukast, zafirlukast montelukast and pranlukast,
sodium channel antagonists such as amiloride, potassium channel antagonists such as bimakalim,
arachidonic acid antagonists such as 2-benzoxazolamine,
histamine receptor antagonists such as epinastine, azelastine, cinnarizine, cetrizine, mizolastine, mequitamium, mequitazine, chlorpheniramine, astemizole, terfenadine, methapyrilene and fenoxfenadine,
antimigrain agents such as ergot alkaloids methisergide, ergotamine, serotonin, sumatriptan, zolmitriptan, cyclandelate etc.,
analgesics such as fentanyl, codeine, morphine, dihydromorphine, buprenorphine, opium, heroin, nalbuphine, pentazocine, oxycodone, tramadol, pethidine, tilidine, methadone, nefopam, dextropropoxyphene, piritramide, etc.,
antiemetics such as bromopride, domperidone, metoclopramide, triethylperazine, trifluoropromazine, meclozine, chlorphenoxamine, dimenhydrinate etc.,
antibiotics such as penicillins (e.g. azlocillin), cephalosporins (e.g. cefotiam or ceftriaxone), carbapenems, monobactams, tetracyclines, aminoglycosides (e.g. streptomycin, neomycin, gentamycin, amikacin or tobramycin), quinolones (e.g. ciprofloxacin), macrolides (e.g. erythromycin), nitroimidazoles (e.g. tinidazol), lincosamide (e.g. clindamycin), glycopeptides (e.g. vancomycin), polypeptides (e.g. bacitracin), mupirocin etc.,
vitamins and free-radical scavengers such as vitamin A, B, C, D or E, catalase, superoxide dismutase, reduced glutathione etc.,
antidiabetics such as glibenclamide, glipizide, gliclazide, glimepiride, troglitazone etc.,
hypnotics such as benzodiazepines, piperidonediones, antihistaminics etc.,
neuroleptics, antidepressants and anticonvulsants such as benzodiazepines, phenothiazines, butyrophenones, sulpiride, hydantoins, barbiturates, succinimides, carbamazepine etc.,
systemically active drugs such as, for example, isosorbide dinitrate, isosorbide mononitrate, diltiazem, xanthines e.g. aminophylline or theophylline, apomorphine and cannabinoids,
antiinflammatory agents,
hormones such as androgens (e.g. testosteron), antioestrogens, calcitonin, parathyrin, somatotropin, oxytocin, prolactin, glucagon, erythropoietin, atriopeptin, melanotropin, thyrotropin, gonadotropin, vasopressin, insulin etc., potency agent such as alprostadil,
cytostatics such as nitrogen mustard derivatives (such as ifosphamide), N-Nitrosourea derivatives (e.g. lomustin), purine and pyrimidine bases antagonists (e.g. fluorouracil), platinum complexes (e.g. carboplatin), anthracyclines (e.g. doxorubicin), podophylline derivatives (e.g. podophyllotoxin).

The mentioned medicaments can optionally be used in the form of their esters, solvates (e.g. hydrates), isomers, enantiomers epimers or racemates and, in the case of acids or bases, as such or in the form of their pharmaceutically acceptable addition salts with organic or inorganic bases or acids.

Preferably the emulsion and microemulsion of the invention comprise an hydrophilic drug.

The optimum amount of active compound in the formulations according to the invention depends on the particular active compound. As a rule, however, aerosol formulations are preferred which contain at least approximately 0.0001 and at most approximately 5% by weight, in particular approximately 0.01 to 3% by weight, of active compound.

For the purposes of the invention, a surfactant with a low hydrophile-lipophile balance of about 3-8 (HLB) is required. The HLB number of a surfactant is a number that expresses the degree of hydrophilicity of the surfactant molecule. In an emulsion, the balance between the hydrophilic and hydrophobic portions of the molecule are important in determining its affinity towards the aqueous and oil phases it is in contact with, and hence how it will behave. At the higher end of the scale the surfactants are hydrophilic and act as solubilising agents, detergents and oil-in-water emulsifiers (HLB = 8-20) (A. T. Florence and D. Attwood, Surfactant Systems: Their Chemistry, Pharmacy and Biology, Chapman and Hall, London, 1983).

In emulsions the concentration of surfactant is in the range of 0.01-1% w/w, whereas for microemulsions the surfactant concentration is approximately 10% w/w. A cosurfactant is generally required e.g. short or long chain alcohols, glycols or polyglycerol derivatives, for the formation of microemulsions. Research into this area of alternative methods of solubilisation has been published by N. Patel et al Drug Delivery to the Lungs IX, London, The Aerosol Society, 160-163 (1998) and M. L. Sommerville and A. J. Hickey, AAPS, 1999.

N. Patel et al work involves the use of fluorinated surfactants with HFA134a propellant. Sommerville and Hickey used model propellants and a lecithin surfactant. As yet no data have been published on the efficiency of these formulations. Other general papers have mentioned anionic AOT (K. A. Johnson and D. O. Shah, J. Colloid Interf. Sci., 107(1), 269-271, 1985; J. L. Fulton and R. D. Smith, US5158704; M. J. Lawrence and G. D. Rees, Adv. Drug Del. Rev., 45, 89-121, 2000) and lecithins (M. J. Lawrence and G. D. Rees, Adv. Drug Del. Rev., 45, 89-121, 2000; P. Schurtenburger et al, J. Colloid Interf. Sci.,156, 43-51, 1993) as popular surfactants for reverse micelle and microemulsion formation but these have not been used for aerosol formulations. Likewise papers have been published on the use of sucrose esters (M. A. Thevenin et al, Int. J. Pharm. 137, 177-186, 1996), alkyl polyglucosides (APGs) (K. Fukuda et al, Colloids and Surfaces B: Biointerfaces 20, 129-135, 2001; L. D. Ryan and E. W. Kaler, Colloids and Surfaces A: Phys. Eng. Aspects 176, 69-83, 2001), and cationic surfactants (M. Olla et al, Colloids and Surfaces A: Phys. Eng. Aspects 160, 23-36, 1999).

A variety of surfactants can be utilised for emulsion formation for the purposes of the application.

They are preferably selected from the group consisting of:
Span 85 (Sorbitan Trioleate), Span 20 (Sorbitan monolaurate),
Aerosol OT (sodium dioctylsulpho-succinate),
DDAB,
Poloxamer known under the trade name Synperonic and the codes: PE/F68,
PE/L61, PE/L64, PE/F127, PE 25R2,
Brij 92/ POE-(2)-oleyl ether,
Epikuron 200,
Tween 80/POE-(20)-sorbitan monolaurate,
ammonium perfluorooctanoate,
alkyl poly(glucosides) known as Plantacare 2000UP/Alkyl(C₈-C₁₆) Glucoside, Sucrose distearate-SP01-C, Sucrose distearate-SP30-C, Sucrose stearate-SP50-C, Sucrose laurate-SP70-C, Sucrose tetrastearate triacetate-A10E-C, Sucrose palmitate-PS750-C,
fluorinated surfactants such as ammonium perfluorooctanoate or known as Zonyl FSN.

Preferred surfactants are Span 85/AOT blends, Synperonics® (Poloxamer) and alkyl poly(glucosides) (APGs).

The preferred cosolvents, when present, particularly useful in microemulsion formulations are lower alkyl (C₁-C₄) alcohols, polyols, polyalkylene glycols and their combinations.

One of the most preferred co-solvent is ethanol.
Other suitable co-solvents are (poly)alkoxy derivatives including polyalkoxy alcohols, in particular 2-(2-ethoxyethoxy)ethanol (available under the trademark Transcutol®).

Further (poly)alkoxy derivatives include polyoxyalkyl ethers and esters, such as polyoxyethylene ethers or esters. The preferred polyoxyethylene ethers and esters are polyoxyethylene alkyl ethers, polyoxyethylene sorbitan fatty acid esters and polyoxyethylene stearates.

As a cosolvent a fatty acid alkyl ester can be also utilized. The preferred fatty acid alkyl esters are ethyl oleate, isopropyl myristate and isopropyl palmitate.

Of the surfactants evaluated, Span 85/AOT blends, Synperonics and alkyl polyglucosides (APGs) formed emulsions. Span 85 is a non-ionic surfactant, also known as sorbitan trioleate (HLB = 1.8). AOT is a twin-tailed anionic surfactant with a very high HLB (HLB = 42), which acts to modify the shape and solubility of the Span 85 at the oil-water interface, by orientating its hydrophobic chain ends into the external oil phase. The Synperonics are triblock copolymers of ethylene oxide and propylene oxide. The ethylene oxides form the hydrophilic chain ends. The name used for the Synperonics describes the structure of the molecule, e.g. L64; L denotes that the surfactant is a liquid, P refers to a paste and F is a solid (flakes). The first number, 6, multiplied by 1800 gives the molecular weight of the hydrophobic portion and the last number, 4, multiplied by 10 gives the percentage of the hydrophilic portion. The structures of Span 85, AOT and Synperonics can be seen in Scheme 1. 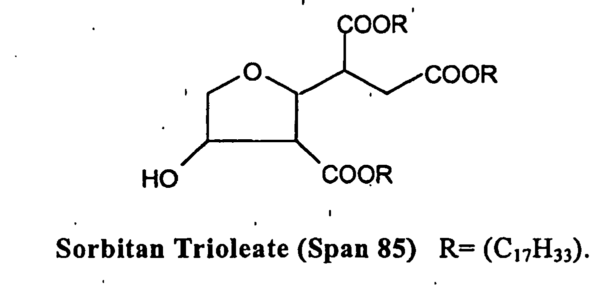 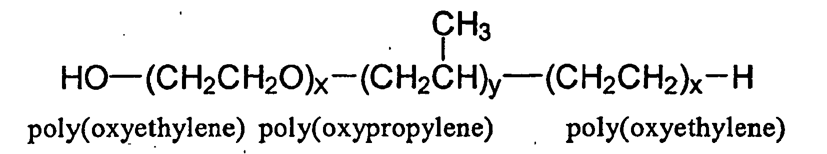 **Synperonics** (diagram shows EPE triblock copolymers: Reverse Synperonics are of opposite conformation, poly(oxypropylene-block-oxyethylene-block-oxypropylene))

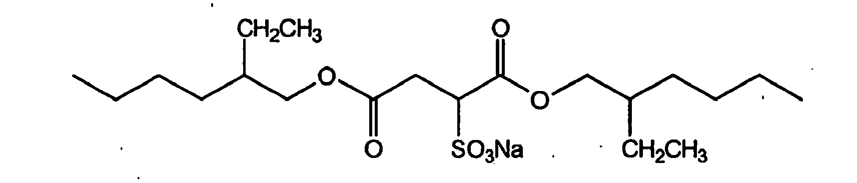

For microemulsion formation, the Synperonics were the particularly preferred surfactant. Low HLB hydrophobic surfactants such as Span 85 and lecithin were used in conjunction with a cosolvent such as ethanol. For slightly more polar HFA formulations, more hydrophilic surfactants, such as polyethylene glycol and derivatives were more suitable.

In some preferred embodiments of the invention, emulsion formulations were based on 0.1-2% surfactant, 1-10% water and hydrofluoroalkane propellant such as HFA227, HFA 134a or their mixtures as the oil phase. The percentages are expressed by weight on the total weight of the formulation. Preferred emulsion formulations are based on 0.1-1% surfactant and 2-8% water.

Even more preferred emulsion formulations were based on 0.1-1% surfactant and 3-6% water, being the 5% water the most preferred concentration.

Microemulsion formulations were based on 1-20% surfactant, 1-30% cosolvent with 1-10% water and hydrofluoroalkane propellant such as HFA227, HFA 134a or their mixtures as the oil phase. Preferred microemulsion formulations were based on 5-15% surfactant, 5-20% cosolvent, 3-6% water and propellant.

Even more preferred microemulsions were based on 5-10% surfactant, 10-20% cosolvent, 5% water. The preferred cosolvent is ethanol.

Characterisation of the emulsions and microemulsions was carried out by a variety of methods.

For the emulsions the most effective method of determining the presence of an oil-in-water or water-in-oil formulation was by centrifugation of the samples and determining the location of the supernatant.

For the microemulsions, the samples have been characterised by dynamic light scattering also known as Photon Correlation Spectroscopy to detect the presence of the droplets of the internal phase. Formulation metering performance was evaluated by determining the emitted dose and drug delivery performance was evaluated via Anderson Cascade Impaction (ACI) measurements according to the method described in Apparatus 2, EP 3^{rd} Edition 1999 supplement, section 2.9.18, Aerosol assessment of fine particles.

Samples were prepared in clear glass formulation vials. The surfactants were added first followed by water then the other components of the formulation were added and the weight of bottle recorded after each addition. Final compositions were calculated as percentage w/w. Valves were crimped onto plastic-coated bottles or anodized aluminium cans before being placed in a sonicator for 5 minutes to allow as much dissolution of surfactants in the water as possible. The propellant was filled through the valve and the final weight of the packaged formulation recorded. In the case of the emulsions the packaged formulations were either shaken vigorously by hand or gently warmed in the hand (sonication where necessary) to induce an emulsion. For the microemulsion formulations the same method was used except the ethanol was added just before crimping and no sonication was required after HFA addition as a clear formulation was obtained immediately. Microemulsion formation occurs immediately on addition of all the formulation components and therefore did not require any additional energy input.

The following tables 1-2 list emulsion formulations prepared with either 25µg/50µl of salbutamol sulphate, oestradiol dipropionate or apomorphine hydrochloride. The surfactants used for these formulations are a Span85/AOT blend and the Synperonics L64. Presence of drug in the formulation had little effect on the emulsion formation.

**Table 1. Span 85/ AOT drug formulations for HFA227. Formulations all contain 5% w/w water and 25 µg/50 µl drug**

| **Oestradiol Dipropionate** | | |
|---|---|---|
| **% Span85** | **% AOT** | **Comment** |
| 0.1 | 0.05 | Emulsion |
| 0.1 | 0.01 | Emulsion |

| **Apomorphine Hydrochloride** | | |
|---|---|---|
| **% Span85** | **% AOT** | **Comment** |
| 0.4 | 0.05 | Emulsion |
| 0.1 | 0.05 | Emulsion |

**Table 2. Synperonic L64 Drug Formulations for HFA227 and HFA134a. Formulations all contain 5% w/w water and 25 µg/50 µl drug**

| **Salbutamol sulphate** | | |
|---|---|---|
| **% L64** | **HFA 227** | **HFA 134a** |
| 1 | Immediate emulsion | Emulsion |
| 0.5 | Immediate emulsion | Emulsion |
| 0.1 | | Emulsion |

| **Oestradiol dipropionate** | | |
|---|---|---|
| **% L64** | **HFA 227** | **HFA 134a** |
| 1 | Immediate emulsion | Emulsion |
| 0.5 | Immediate emulsion | Emulsion |
| 0.1 | | Emulsion |

| **Apomorphine Hydrochloride** | | |
|---|---|---|
| **% L64** | **HFA 227** | **HFA 134a** |
| 1 | Emulsion | Emulsion |
| 0.5 | Emulsion | Emulsion |
| 0.1 | Emulsion | Emulsion |

Centrifugation of the emulsions showed the internal phase coalescing and then rising up or down depending on the difference in density with the external phase. Results suggest that Span 85/AOT formulations comprise water as the less dense internal phase and conversely, the propellant forming the denser internal phase for the Synperonic. Therefore Span 85/AOT surfactant blend forms a water-in-oil emulsion and the Synperonic PE/L64 form oil-in-water emulsions.

The following table 3 lists the various microemulsion formulations prepared with Synperonic L64, AOT, and the fluorinated surfactant Zonyl FSN. L64 and Zonyl FSN produced clear systems.

**Table 3. Microemulsion formulations. All formulations contain 5% w/w water.**

| **Surfactant** | **%** | **HFA** | **EtOH %** | **Comment** |
|---|---|---|---|---|
| Syn. PE/L64 | 10 | 227 | 20 | Clear microemulsion |
| Syn. PE/L64 | 10 | 227 | 10 | Cloudy solution, thin top layer |
| Syn. PE/L64 | 5 | 227 | 20 | Cloudy solution, thin top layer |
| Syn. PE/L64 | 5 | 227 | 10 | Cloudy solution, thin top layer |
| AOT | 10 | 227 | 20 | Slight cloud, one apparent phase |
| Zonyl FSN | 10 | 227 | 20 | Clear yellow, one apparent phase |

Photon Correlation Spectroscopy (PCS) characterization of the Synperionic L64 based microemulsions was carried out and the results are shown in Table 4.

**Table 4. Photon Correlation Spectroscopy (PCS) results showing microemulsion particle size.**

| **L64 (%)** | **EtOH (%)** | **Water (%)** | **Salbutamol sulphate (µg/dose)** | **Microemulsion particle size (nm)** |
|---|---|---|---|---|
| 10 | - | - | - | 7.5 |
| 10 | 20 | - | - | 3.0 |
| 10 | 20 | 3 | - | 4.5 |
| 10 | 20 | 5 | - | 8.0 |
| 13.3 | 26.6 | 7.5 | - | 6.6 |
| 13.3 | 26.6 | 10 | - | 19.6 |
| 10 | 20 | 70 | - | 20.0 |
| 10 | 20 | 5 | 25 | 10.0 |

One of the tested formulations contained 25 µg of salbutamol sulphate per 50 µl metering volume.

Cascade impaction studies were carried out on salbutamol sulphate microemulsion formulations using an Andersen cascade impactor (ACI). The ACI was operated at a flow rate of 28.3 ± 2 1 min⁻¹. Formulations were discharged into the ACI through either a 0.42 mm orifice diameter commercially available or a prototype actuator. Drug deposition was determined using a RP-HPLC method.

Drug deposition was mainly in the throat.

The formulation of Tables 3 and 4 could be suitable for aerosols for the oral and nasal delivery.

For pulmonary delivery a larger amount of respirable particles (i.e. ≤ 4.7 µm) aerodynamic diameter as measured by ACI are required.

The selection of surfactant or surfactant mixture, their concentration, the modulation of the ratios surfactant/cosolvent, surfactant/water, surfactant/cosolvent/water and the selection of the actuator orifice diameter of the pMDI could allow to improve both the metering performance and the particle size distribution with an increased fine particle fraction (respirable fraction) of the aerosol.

## Claims

1. An emulsion or microemulsion pharmaceutical aerosol formulation comprising: a) an effective amount of a medicament; b) a hydrofluoroalkane propellant selected from the group of HFA 134a, HFA 227 and their mixtures; c) one or more surfactants; d) water and e) optionally a cosolvent.

2. An aerosol formulation according to claim 1 wherein the surfactant is Span 85 (Sorbitan Trioleate), Span 20 (Sorbitan monolaurate), Aerosol OT (sodium dioctylsulpho-succinate), DDAB, Poloxamer known under the trade name Synperonic and the codes: PE/F68, PE/L61, PE/L64, PE/F127, PE 25R2, Brij 92/ POE-(2)-oleyl ether, Epikuron 200, Tween 80/POE-(20)-sorbitan monolaurate, ammonium perfluorooctanoate, alkyl poly(glucosides) known as Plantacare 2000UP/Alkyl(C8-C16) Glucoside, Sucrose distearate-SP01-C, Sucrose distearate-SP30-C, Sucrose stearate-SP50-C, Sucrose laurate-SP70-C, Sucrose tetrastearate triacetate-A10E-C, Sucrose palmitate-PS750-C, fluorinated surfactants such as ammonium perfluorooctanoate or known as Zonyl FSN.

3. An aerosol formulation according to claims 1 and 2 wherein the surfactants are Span 85/AOT blends, Synperonics® (Poloxamer) and alkyl poly(glucosides) (APGs).

4. An aerosol formulation according to claims 1-3 wherein the formulation is an emulsion and the surfactant concentration is from 0.1 to 2 w/w % of the total formulation.

5. An aerosol formulation according to claim 4 wherein the surfactant concentration is from 0.1 to 1 w/w % of the total formulation.

6. An aerosol formulation according to claims 1-3 wherein the formulation is a microemulsion and the surfactant concentration is from 1 to 20 w/w % of the total formulation.

7. An aerosol formulation according to claim 6 wherein the surfactant concentration is from 5 to 15 w/w% of the total formulation.

8. An aerosol formulation according to claims 1-3 and 6-7 wherein the cosolvent is selected from lower alkyl (C1-C4) alcohols, polyols, polyalkylene glycols and their combination; (poly)alkoxy derivatives including polyalkoxy alcohols, in particular 2-(2-ethoxyethoxy)ethanol (available under the trademark Transcutol®), polyoxyalkyl ethers and esters, such as polyoxyethylene ethers or esters, a fatty acid alkyl ester such as ethyl oleate, isopropyl myristate and isopropyl palmitate.

9. An aerosol formulation according to claim 8 wherein the cosolvent is ethanol.

10. An aerosol formulation according to claims 8 and 9 wherein the cosolvent concentration is from 1 to 30 w/w % of the total formulation.

11. An aerosol formulation according to claim 10 wherein the cosolvent concentration is from 5 to 20 w/w % of the total formulation.

12. An aerosol formulation according to claims 1-11 wherein the water concentration is from 1 to 10 w/w % of the total formulation.

13. An aerosol formulation according to claim 12 wherein the water concentration is from 2 to 8 w/w % of the total formulation.

## Patentansprüche

1. Pharmazeutische Emulsions- oder Mikroemulsions-Aerosolformulierung, umfassend:
a) eine wirksame Menge eines Medikaments; b) ein Hydrofluoralkan-Treibmittel, ausgewählt aus der Gruppe aus HFA 134a, HFA 227 und deren Gemischen; c) ein oberflächenaktives Mittel oder mehrere oberflächenaktive Mittel; d) Wasser und e) gegebenenfalls ein Co-Lösungsmittel.

2. Aerosolformulierung nach Anspruch 1, wobei das oberflächenaktive Mittel Span 85 (Sorbitantrioleat), Span 20 (Sorbitanmonolaurat), Aerosol OT (Natriumdioctylsulfosuccinat), DDAB, Poloxamer, bekannt unter der Handelsbezeichnung Synperonic und den Codenamen: PE/F68, PE/L61, PE/L64, PE/F127, PE 25R2, Brij 92/POE-(2)-oleylether, Epikuron 200, Tween 80/POE-(20)-sorbitanmonolaurat, Ammoniumperfluoroctanoat, Alkylpoly(glucoside), bekannt als Plantacare, 2000UP/Alkyl(C8-C16)glucosid, Saccharosedistearat-SP01-C, Saccharosedistearat-SP30-C, Saccharosestearat-SP50-C, Saccharoselaurat-SP70-C, Saccharosetetrastearattriacetat-A10E-C, Saccharosepalmitat-PS750-C, fluorierte oberflächenaktive Mittel wie Ammoniumperfluoroctanoat oder bekannt als Zonyl FSN ist.

3. Aerosolformulierung nach Anspruch 1 und 2, wobei die oberflächenaktiven Mittel Span 85/AOT-Mischungen, Synperonics^{®} (Poloxamer) und Alkylpoly(glucoside) (APGs) sind.

4. Aerosolformulierung nach den Ansprüchen 1 bis 3, wobei die Formulierung eine Emulsion ist und die Konzentration des oberflächenaktiven Mittels 0,1 bis 2 Gew.-% der Gesamtformulierung ist.

5. Aerosolformulierung nach Anspruch 4, wobei die Konzentration des oberflächenaktiven Mittels 0,1 bis 1 Gew.-% der Gesamtformulierung ist.

6. Aerosolformulierung nach den Ansprüchen 1 bis 3, wobei die Formulierung eine Mikroemulsion ist und die Konzentration des oberflächenaktiven Mittels 1 bis 20 Gew.-% der Gesamtformulierung ist.

7. Aerosolformulierung nach Anspruch 6, wobei die Konzentration des oberflächenaktiven Mittels 5 bis 15 Gew.-% der Gesamtformulierung ist.

8. Aerosolformulierung nach den Ansprüchen 1 bis 3 und 6 bis 7, wobei das Co-Lösungsmittel unter Niederalkyl(C1-C4)-alkoholen, Polyolen, Polyalkylenglykolen und ihrer Kombination; (Poly)alkoxyderivaten, einschließlich Polyoxyalkoholen, insbesondere 2-(2-Ethoxyethoxy)ethanol (unter der Marke Transcutol^{®} erhältlich), Polyoxyalkylethern und -estern, z.B. Polyoxyethylenethern oder -estern, einem Fettsäurealkylester, z.B. Ethyloleat, Isopropylmyristat und Isopropylpalmitat, ausgewählt ist.

9. Aerosolformulierung nach Anspruch 8, wobei das Co-Lösungsmittel Ethanol ist.

10. Aerosolformulierung nach Anspruch 8 und 9, wobei die Konzentration des Co-Lösungsmittels 1 bis 30 Gew.-% der Gesamtformulierung ist.

11. Aerosolformulierung nach Anspruch 10, wobei die Konzentration des Co-Lösungsmittels 5 bis 20 Gew.-% der Gesamtformulierung ist.

12. Aerosolformulierung nach den Ansprüchen 1 bis 11, wobei die Wasserkonzentration 1 bis 10 Gew.-% der Gesamtformulierung ist.

13. Aerosolformulierung nach Anspruch 12, wobei die Wasserkonzentration 2 bis 8 Gew.-% der Gesamtformulierung ist.

## Revendications

1. Formulation aérosol pharmaceutique en émulsion ou microémulsion comprenant: a) une quantité efficace d'un médicament; b) un propulseur hydrofluoroalcane choisi dans le groupe de HFA 134a, HFA 227 et leurs mélanges ; c) un ou plusieurs agents tensioactifs ; d) de l'eau et e) facultativement un cosolvant.

2. Formulation aérosol selon la revendication 1, dans laquelle l'agent tensioactif est le Span 85 (trioléate de sorbitane), le Span 20 (monolaurate de sorbitane), l'Aerosol OT (dioctylsulfosuccinate de sodium), le DDAB, un Poloxamer connu sous le nom de commerce Synperonic et les codes : PE/F68, PE/L61, PE/L64, PE/F127, PE 25R2, Brij 92/POE-(2)-éther oléylique, Epikuron 200, Tween 80/POE-(20)-monolaurate de sorbitane, le perfluorooctanoate d'ammonium, les poly(glucosides) d'alkyle connus sous le nom de Plantacare 2000UP/Alkyl(C₈-C₁₆) Glucoside, le distéarate de saccharose-SP01-C, le distéarate de saccharose-SP30-C, le stéarate de saccharose-SP50-C, le laurate de saccharose-SP70-C, le tétrastéarate triacétate de saccharose-A10E-C, le palmitate de saccharose-PS750-C, les agents tensioactifs fluorés tels que le perfluorooctanoate d'ammonium ou connu sous le nom de Zonyl FSN.

3. Formulation aérosol selon les revendications 1 et 2, dans laquelle les agents tensioactifs sont les mélanges Span 85/AOT, le Synperonics® (Poloxamer) et les poly(glucosides) d'alkyle (APG).

4. Formulation aérosol selon les revendications 1-3, dans laquelle la formulation est une émulsion et la concentration en agent tensioactif est de 0,1 à 2 % p/p de la formulation totale.

5. Formulation aérosol selon la revendication 4, dans laquelle la concentration en agent tensioactif de 0,1 à 1 % p/p de la formulation totale.

6. Formulation aérosol selon les revendications 1 à 3, dans laquelle la formulation est une microémulsion et la concentration en agent tensioactif est de 1 à 20 % p/p de la formulation totale.

7. Formulation aérosol selon la revendication 6, dans laquelle la concentration en agent tensioactif est de 5 à 15 % p/p de la formulation totale.

8. Formulation aérosol selon les revendications 1-3 et 6-7, dans laquelle le cosolvant est choisi parmi les alcools alkyliques inférieurs en (C₁-C₄), les polyols, les polyalkylèneglycols et leur combinaison ; les dérivés poly(alcoxylés) incluant les alcools polyalcoxylés, en particulier le 2-(2-éthoxyéthoxy)éthanol (disponible sous le nom de commerce Transcutol®), les éthers et esters polyoxyalkyliques tels que les éthers ou esters polyoxyéthyléniques, un ester alkylique d'acide gras tel que l'oléate d'éthyle, le myristate d'isopropyle et le palmitate d'isopropyle.

9. Formulation aérosol selon la revendication 8, dans laquelle le cosolvant est l'éthanol.

10. Formulation aérosol selon les revendications 8 et 9, dans laquelle la concentration en cosolvant est de 1 à 30 % p/p de la formulation totale.

11. Formulation aérosol selon la revendication 10, dans laquelle la concentration en cosolvant est de 5 à 20 % p/p de la formulation totale.

12. Formulation aérosol selon les revendications 1-11, dans laquelle la concentration en eau est de 1 à 10 % p/p de la formulation totale.

13. Formulation aérosol selon la revendication 12, dans laquelle la concentration en eau est de 2 à 8 % p/p de la formulation totale.
